Europäisches Patentamt

European Patent Office

Office européen des brevets

(11) Veröffentlichungsnummer: **0 274 111 B1**

# (12) EUROPÄISCHE PATENTSCHRIFT

(45) Veröffentlichungstag der Patentschrift: 30.10.91

(51) Int. Cl.5: **B01D 53/14,** C07D 201/16

(21) Anmeldenummer: 87119007.0

(22) Anmeldetag: 22.12.87

(54) **Verfahren zur Entfernung von Benzol und Toluol aus Abgasen.**

(30) Priorität: 07.01.87 DE 3700247

(43) Veröffentlichungstag der Anmeldung:
13.07.88 Patentblatt 88/28

(45) Bekanntmachung des Hinweises auf die
Patenterteilung:
30.10.91 Patentblatt 91/44

(84) Benannte Vertragsstaaten:
BE CH DE ES FR GB IT LI NL

(56) Entgegenhaltungen:
EP-A- 0 043 924
EP-A- 0 084 319
FR-A- 2 203 818
US-A- 4 145 192

CHEMICAL ABSTRACTS, Band 84, 1976, Seite
120, Zusammenfassung Nr. 92049h, Columbus, Ohio, US; K. WEHNER:
"N-Alkyl-e-caprolactams as new selective
extracting agents for technical separation
processes"

(73) Patentinhaber: **BASF Aktiengesellschaft**
**Carl-Bosch-Strasse 38**
**W-6700 Ludwigshafen(DE)**

(72) Erfinder: **Brand, Uwe**
**Rheingoldstrasse 12**
**W-6840 Lampertheim(DE)**
Erfinder: **de Decker, Emile**
**Josef Copstraat 4 (Bus 1)**
**B-2710 Hoboken(BE)**
Erfinder: **Deuker, Ernst, Dr.**
**Westring 31**
**W-6718 Grünstadt(DE)**
Erfinder: **Fuchs, Hugo, Dr.**
**Egellstrasse 28**
**W-6700 Ludwigshafen(DE)**
Erfinder: **Kartte, Klaus, Dr.**
**Stettiner Strasse 1**
**W-6711 Beindersheim(DE)**
Erfinder: **Neubauer, Gerald, Dr.**
**Mozartstrasse 24**
**W-6940 Weinheim(DE)**
Erfinder: **Oostvogels, Jozef**
**Cederlaan 15**
**B-2120 Schoten(BE)**

## Beschreibung

Bei einer Reihe von chemischen Umsetzungen, Destillationen, Extraktionen oder Verdampfungsvorgängen fallen Abgase an, die flüchtige Lösungsmittel enthalten. Solche lösungsmittelhaltige Abgase können nicht ohne weitere Reinigung in die Atmosphäre abgegeben werden. Eine Möglichkeit der Reinigung solcher Abgase besteht darin, daß man die Lösungsmittel durch Behandeln mit Aktivkohle absorbiert. Es ist jedoch sehr aufwendig, hieraus wieder die Lösungsmittel zu gewinnen. Deshalb werden die Absorptionsmittel wie Aktivkohle zusammen mit dem Lösungsmittel verbrannt. Nach einer anderen Arbeitsweise werden solche lösungsmittelhaltigen Abgase über eine Fackel geleitet und die darin enthaltenen Lösungsmittel verbrannt. Hierbei sind die Lösungsmittel ebenfalls verloren und im Falle von Chlorkohlenwasserstoffen entsteht unerwünschter Chlorwasserstoff.

Aus der EP-A- 84 319 ist bekannt, Benzol oder Toluol aus Abgasen durch Waschen mit Gemischen aus Glykol, N-Methylcaprolactam und Wasser, insbesondere einem Gemisch, das zum überwiegenden Teil aus Glykol, 2 bis 10 Gew.-% N-Methylcaprolactam und 0,1 bis 1 Gew.-% Wasser besteht, zu entfernen.

Es war deshalb die technische Aufgabe gestellt, mit Wasser nicht mischbare Lösungsmittel aus solches enthaltenden Abgasen auf einfache Weise wirksam zu entfernen, wobei die Lösungsmittel in wiedergewinnbarer Form anfallen.

Diese Aufgabe wird gelöst in einem Verfahren zur Entfernung von Benzol oder Toluol aus solches enthaltenden Abgasen, die bei der Extraktion von Rohlactam anfallen, wobei man die Benzol oder Toluol enthaltenden Abgase mit 5 bis 40 Gew.-% Wasser enthaltendem Caprolactam wäscht und die gelöstes Benzol oder Toluol enthaltende wäßrige Caprolactamlösung in die Extraktion von Rohlactam zurückführt.

Das neue Verfahren hat den Vorteil, daß es einfach durchführbar ist und die Lösungsmittel wirkungsvoll aus den Abgasen entfernt werden. Ferner hat das neue Verfahren den Vorteil, daß die Lösungsmittel in wiedergewinnbarer Form anfallen.

In der Regel enthalten die behandlungsbedürftigen Abgase 5 bis 1000 ppm, insbesondere 10 bis 1000 ppm Benzol oder Toluol.

Erfindungsgemäß werden die behandlungsbedürftigen Abgase mit Caprolactam mit einem Wassergehalt von 5 bis 40 Gew.-% Wasser gewaschen. Geeignet ist auch Rohlactam, wie es nach Neutralisation des Umlagerungsgemisches aus der Beckmann'schen Umlagerung nach Abtrennen der wäßrigen Phase anfällt.

Die Wäsche erfolgt zweckmäßig in üblichen Vorrichtungen, wie sie für die Gaswäsche verwendet werden, z.B. Füllkörperkolonnen, Sieb- oder Glockenbodenkolonnen. In der Regel erfolgt die Wäsche im Gegenstrom, wobei man am Kopf der Kolonne Wasser enthaltendes Caprolactam aufgibt und unten behandlungsbedürftiges Abgas einleitet. Am Kopf der Kolonne wird gereinigtes Abgas entnommen. Vorteilhaft wendet man je Nm$^3$ behandlungsbedürftiges Abgas 50 bis 5000 kg Wasser enthaltendes Caprolactam an.

Vorteilhaft haben die zu behandelnden Abgase eine Temperatur von 10 bis 100° C und das zur Wäsche verwendete Wasser enthaltende Caprolactam eine Temperatur von 10 bis 80° C.

Für das Verfahren nach der Erfindung verwendet man zur Entfernung Benzol oder Toluol enthaltende Abgase, die bei der Extraktion von Caprolactam aus Rohlactam anfallen. Es handelt sich hierbei um Inertgase mit einem Gehalt von 10 bis 5000 ppm an Benzol oder Toluol. Die behandlungsbedürftigen Abgase werden mit einer Temperatur von 10 bis 100° C unten in eine Kolonne eingeleitet und am oberen Teil der Kolonne Rohlactam mit einem Wassergehalt von 10 bis 40 % und einer Temperatur von 20 bis 80° C zugeführt. Es hat sich auch bewährt, wenn am oberen Ende der Kolonne ein Glockenboden angeordnet ist, der zusätzlich mit Wasser oder einer Lösung von 1 bis 70 % Caprolactam in Wasser in einer Menge von 50 bis 1000 kg je Nm$^3$ Abgas zusätzlich beaufschlagt wird.

Die nunmehr Benzol oder Toluol enthaltende wäßrige Caprolactamlösung kann ganz oder partiell im Kreis geführt werden, wobei sich die Wiederverwendung nach dem Gehalt an Lösungsmittel im Abgas richtet und wird wieder der Extraktion zugeführt.

Das Verfahren nach der Erfindung sei an folgenden Beispielen veranschaulicht.

Beispiel 1

10 m$^3$/h eines bei der Extraktion des Lactams aus dem Lactam-Öl mit Benzol anfallenden Inertgases, mit einem Benzolgehalt von 100 ppm, werden in den unteren Teil einer Füllkörperkolonne mit folgenden Kenngrößen geleitet:
Höhe          6 000 mm
Durchmesser          300 mm
Füllkörper Pallringe          25 mm Schütthöhe (2 × 1 500 mm)

Nach der Füllkörperschicht von 2 × 1 500 mm werden am oberen Kolonnenende ca. 4 m$^3$/h eines Lactam-Öls mit 69,5 % Caprolactam, 30 % Wasser und 0,5 % Ammonsulfat aufgegeben. Das Lactam-Öl hatte eine Temperatur von 30° C. Die Kolonne hat im oberen Teil einen Durchmesser von 200 mm und besitzt einen zusätzlichen Glockenboden, der mit ca. 0,4 m$^3$/h Wasser von 20° C beaufschlagt wird. Das aus der Kolonne entweichende Abgas enthält nun 3 ppm Benzol. Das am unteren Ende der Kolonne austretende Lactam-Öl mit dem gelösten Benzol wird in die Extraktionsstufe der Lactamreinigung zurückgeführt.

Anstelle des Glockenbodens kann der obere Kolonnenteil, vom Durchmesser 200 mm, auch mit einer Füllkörperschicht von 300 mm Pallringen der Größe 15 mm, gefüllt sein.

Beispiel 2

Wie in Beispiel 1, werden 10 m³/h des 100 ppm Benzol enthaltenden Inertgases in den unteren Teil der Kolonne geleitet. Die am oberen Ende der Kolonne aufgegebene wäßrige Lactamlösung enthält 85 % Caprolactam und 15 % Wasser. Die Menge beträgt 5 m³/h, die Temperatur 40° C. Der obere Boden wird mit 0,4 m³/h Wasser, welches ca. 2 % Caprolactam enthält, beaufschlagt.

Die am unteren Ende der Kolonne austretende wäßrige Lactamlösung mit dem gelösten Benzol wird in die Extraktionsstufe zurückgeführt. Das Abgas enthält 4 ppm Benzol.

**Patentansprüche**

1. Verfahren zur Entfernung von Benzol oder Toluol aus solches enthaltenden Abgasen, die bei der Extraktion von Caprolactam aus Rohlactam anfallen, dadurch gekennzeichnet, daß man die Benzol oder Toluol enthaltenden Abgase mit 5 bis 40 Gew.-% Wasser enthaltendem Caprolactam wäscht und die gelöstes Benzol oder Toluol enthaltende wäßrige Caprolactamlösung in die Extraktion von Rohlactam zurückführt.

2. Verfahren nach Anspruch 1, dadurch gekennzeichnet, daß man Rohlactam als Waschmittel verwendet.

3. Verfahren nach den Ansprüchen 1 und 2, dadurch gekennzeichnet, daß man je Nm³ Abgas 50 bis 5000 kg Wasser enthaltendes Caprolactam verwendet.

**Claims**

1. A process for removing benzene or toluene from an offgas containing the same obtained in the extraction of caprolactam from crude lactam, which comprises scrubbing the benzene- and toluene-containing offgas with caprolactam containing from 5 to 40 % by weight of water and recycling the aqueous caprolactam solution which contains dissolved benzene or toluene into the crude lactam extraction stage.

2. A process as claimed in claim 1, wherein the scrubbing agent used is crude lactam.

3. A process as claimed in claim 1 or 2, wherein from 50 to 5000 kg of water-containing caprolactam is used per standard m³ of offgas.

**Revendications**

1. Procédé pour éliminer le benzène ou le toluène contenu dans des gaz résiduaires produits à l'extraction du caprolactame à partir du lactame brut, caractérisé par le fait qu'on lave les gaz résiduaires contenant le benzène ou le toluène par du caprolactame contenant 5 à 40 % en poids d'eau et on recycle la solution aqueuse de caprolactame contenant le benzène ou le toluène dissous à l'extraction du lactame brut.

2. Procédé selon la revendication 1, caractérisé par le fait que l'on utilise du lactame brut en tant que liquide de lavage.

3. Procédé selon la revendication 1 et 2, caractérisé par le fait que l'on utilise de 50 à 5000 kg de caprolactame aqueux par m³ normal de gaz résiduaires.